# EUROPEAN PATENT SPECIFICATION

(11) **EP 3 939 641 B1**
(45) Date of publication and mention of the grant of the patent: **26.10.2022**
(21) Application number: 20721666.4
(22) Date of filing: 09.03.2020
(51) Int. Cl.: A61M 16/04, A61B 17/12

(54) **OROPHARYNGEAL OBTURATION DEVICE**
OROPHARYNGEALE VERSCHLUSSVORRICHTUNG
DISPOSITIF DE TAMPONNADE OROPHARYNGÉE

(30) Priority: 11.03.2019 ES 201900039
(43) Date of publication of application: 19.01.2022
(73) Proprietor: Fundación Incliva, 46010 Valencia (ES)
(72) Inventor: PUCHE TORRES, Miguel, 46010 Valencia (ES)
(74) Representative: San Martin Alarcia, Esther
(86) International application number: PCT/ES2020/070163
(87) International publication number: WO 2020/183043

(56) References cited:
- WO-A1-94/19050
- WO-A1-2014/149696
- US-A1- 2014 163 527

## Description

### OBJECT OF THE INVENTION

The object of the present invention is a new device designed for blocking the respiratory airways and the digestive tract of a patient during a surgical procedure in the oral, maxillofacial or craniofacial areas with nasotracheal intubation.

### BACKGROUND OF THE INVENTION

Surgical procedures in the oral cavity, or generally speaking in the facial area, frequently require nasotracheal intubation allowing for adequate pulmonary ventilation during the anaesthetic procedure, while at the same time leaving room in the oral cavity for carrying out the surgical procedure. Therefor, the anaesthetist introduces a tube through the nasal cavity which, passing through the rhynopharynx, the oropharynx and the hypopharynx, reaches the trachea through the vocal cords. Said tube has an inflatable balloon for pneumatically sealing the trachea, allowing for the supply of oxygen and anaesthetic gases to the patient.

During these procedures, the patient secrets saliva, bleeds due to the surgical procedure, emits purulent liquids in case of draining during an infectious process, emits products caused by rinsing with saline solution and other surgical procedure debris, and even foreign bodies may accidentally fall (osteosynthesis screws, small size instruments, etc.). Since the patient is in the supine position, these substances and foreign bodies may accidentally fall towards the hypopharynx and be swallowed, thereby causing harmful consequences for the patient. Thus, there exist a necessity to protect the respiratory airways and digestive tract of the patient by means of nasotracheal intubation and pharyngeal obturation.

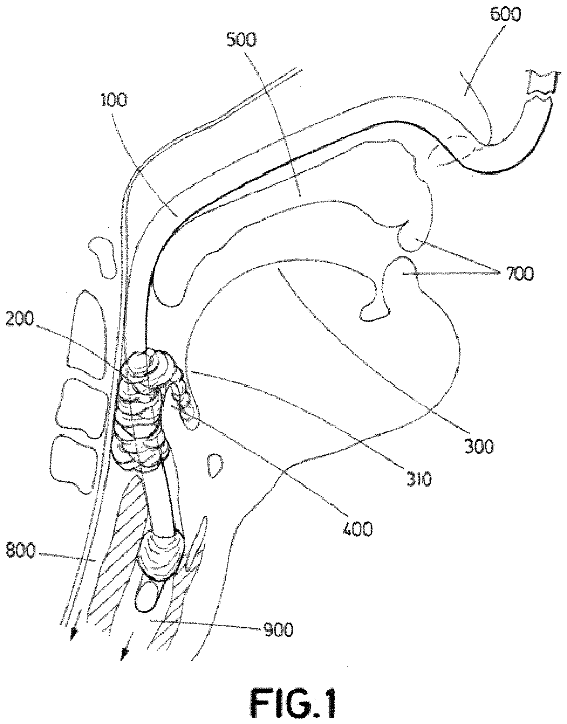

For years, the solution for preventing the passage of these liquids and/or foreign bodies to the digestive tract was the provision of a oropharyngeal obturation consisting generally of a gauze bandage soaked in saline solution and subsequently wringed out (moistened) in the oropharynx and hypopharynx once the nasotracheal tube is appropriately positioned and fixed and the sealing balloon is inflated. For positioning this conventional gauze obturation, the anaesthetist usually employs a Magill clamp and a laryngoscope or video laryngoscope. This solution is shown in Fig. 1, where the anatomy of a patient is shown schematically, including: the tongue (300), the back of the tongue (310), the epiglottis (400), the palate (500), the nose (600), the lips (700), the digestive tract (800), the respiratory airways (900). In this context, the gauze bandage (200) wound around the nasotracheal tube (100) in the area corresponding to the oropharynx of the patient is shown.

During the subsequent surgical procedure, in principle all the liquids not aspirated by the surgical assistant will dampen the abovementioned gauze bandage. Similarly, any foreign body that may fall towards the pharynx would be trapped on the gauze bandage and ideally should be extracted when said bandage is removed. However, the gauze bandage does not always achieve these objects, mainly for the following reasons:
1. The selection of a size and positioning method for the gauze bandage are not objectively determined; they depend on the skill of the health professional carrying out the procedure.
   Sometimes, a large gauze bandage is provided in order to ensure that the surgical site is as hermetic as possible. However, a too large bandage may apply excessive pressure on the hypopharynx mucosa, thus causing discomfort to the patient during the post-operative period.
   On the contrary, sometimes the gauze bandage size is small in order to avoid causing damage to the patient. However, a too small gauze bandage may not render a hermetic sealing of the surgical site, such that the passage of blood and other elements towards the digestive tract of the patient is not appropriately prevented.
2. The gauze bandage is positioned by successively introducing sections or segments aided by the Magill clamp, these sections or segments being pushed towards the hypopharynx. During this procedure, undesirably causing lesions in the pharyngeal mucosa due to abrasion by rubbing against the Magill clamp is not exceptional.
3. The worst and most feared complication caused by the conventional oropharyngeal obturation is that, once the procedure is finished, the obturation is forgotten inside. Indeed, since the obturation is not visible, it can sometimes be forgotten when the procedure is finished. In a best-case scenario, the patient swallows it completely and it is subsequently extracted by endoscopy. In a worst-case scenario, the patient asphyxiates due to the obstruction of the respiratory airways.

In short, there is a need in the art for an oropharyngeal obturator that solves the aforementioned drawbacks.

Document US2014/163527 A1 discloses a device for targeted delivery of a substance to an airway that may include a conduit and at least two applicators. The conduit may include a proximal end and a bifurcated distal portion having two distal ends. Each applicator may be coupled with one of the distal ends of the conduit and may be configured to direct the substance out of the applicator toward one of two sides of an airway.

Document WO2014/149696 A1 discloses an endotracheal tube having an absorbent device attached so that the absorbent device is able to absorb and block any procedure produced debris. The absorbent device has strings that are attached to a device around an endotracheal tube so that the absorbent device is properly dislodged at the end of the procedure.

Document WO94/19050 A1 discloses a surgical sponge for use in the larynx of a throat comprising a porous cellular absorbent sponge material preformed in a compressed rigid member which retains its compressed small volume condition. The surgical sponge is constructed of cellular absorbent sponge material which wicks up fluid from the body to expand from its compressed condition to a predetermined larger size to form an arcuate elongated body with flat end surfaces and a "C" shaped cross section defining a concave channel, at least one of the flat end surfaces being coated with a fluid impervious layer which prevents the flow of fluid therethrough. The sponge material is impregnated with a radiopaque material such as barium sulfate, and is provided with an attaching cord allowing removal of the surgical sponge from the patient. The attaching cord includes a clip to prevent the surgical sponge from being swallowed.

The invention is defined by the appended claims.

### DESCRIPTION OF THE INVENTION

The present application describes an oropharyngeal obturator that solves the aforementioned drawbacks that dispenses with the need to use an inflating pneumatic system like the one used in some known devices. Indeed, the device of the present invention may fold for reducing its volume only due to its elasticity and/or shape memory characteristics, such that installation in the respiratory airway adjacent the oropharynx is carried out fast and conveniently. Further, the device of the invention has a safety element preventing it to be forgotten inside the respiratory airway of the patient once the procedure is finished. An introductor is further disclosed for facilitating even more the installation of the device.

In the present document, terms such as "front", "rear", "up", "low" and similar ones must be interpreted according to the natural position of the device in the respiratory airway of a patient that is in a vertical position. That is, using usual medical terminology, the term "up" refers to a cranial direction, the term "low" refers to an inferior direction, the term "front" refers to an anterior direction, and the term "rear" refers to a posterior direction.

The invention is directed to an oropharyngeal obturation device configured for blocking the respiratory airway of a patient adjacent the oropharynx during a surgical procedure with nasotracheal intubation. This device mainly comprises the following parts: an obturator element, an attachment element, and a safety element. Now, each of these elements is disclosed in more detail.

### a) Obturator element

The purpose of the obturator element is to block the oropharynx and the hypopharynx of the patient around the nasotracheal tube for preventing the passage of fluids or foreign elements. Therefor, the obturator element has a shape comprising a lower portion and an upper portion.

The lower portion comprises two parallel lobular cavities shaped as two bottom-closed sacs, and is configured for adapting to the anatomy of the front portion of the oropharynx of the patient such that it abuts on the tongue side with the epiglottis housed in the gap between both lobular cavities.

The upper portion comprises a rear wall protruding from rear edges of the lobular cavities, and is configured for adapting to the anatomy of the rear portion of the oropharynx of the patient such that it abuts on the rear wall of the oropharynx.

Thanks to this configuration, the obturator element, once installed in the respiratory airway of the patient adjacent the oropharynx, adapts perfectly to the anatomy of the patient and, thus, achieves an essentially hermetic sealing of the respiratory airway around the nasotracheal tube, therefore preventing the passage of fluids or foreign elements.

Further, the obturator element has shape memory, such that it can fold during the procedure for introducing the device and subsequently recover its shape once in the oropharyngeal area of the patient. The obturator element can be folded simply by applying compression with a clamp or any other suitable surgical instrument employed for the introduction thereof in the respiratory airway of the patient. Thus, not only introduction of the device through the mouth in the respiratory airway of the patient is facilitated, but also an effective sealing of the respiratory airway is promoted, since it expands for recovering its shape once the position of the oropharynx is reached.

In principle, the obturator element may have any configuration provided in can be folded and, subsequently, recover its original shape designed for adapting to the respiratory airway of the patient adjacent the oropharynx. However, in a particularly preferred embodiment of the present invention, the obturator element is made of an elastic material provided with a peripheric reinforcement along an external edge thereof having a lower elasticity than that of the rest of the obturator element. That is, the peripheric element is somewhat more rigid than the surface of the obturator element such that, once the obturator element as a whole is folded, the peripheric element ensures recovery of the original shape once the force maintaining it in the folded condition disappears when released in the oropharyngeal area.

The obturator element may be made of any material complying with the above requirements and being also compatible for medical use. Normally a hypoallergenic material free of latex, such as plastic or silicone, is used.

### b) Attachment element

The purpose of the attachment element is to ensure a firm connection between the device of the invention and the nasotracheal tube installed in the patient. Therefore, at least a attachment element is connected to the obturator element and configured for being coupled to the nasotracheal tube adjacent the oropharynx of the patient. Normally, the attachment element is made as a single part with the obturator element, thus preventing the possible detachment of a part. In any case, it would be possible that the connection between the obturator element and the attachment element to be dismountable provided it is sufficiently safe in this respect.

In principle, the attachment element could be designed in a number of different ways provided it allows for a dismountable connection between the device of the invention and the nasotracheal tube. However, in a particularly preferred embodiment of the invention, the attachment element comprises at least a clamp configured for coupling to the nasotracheal tube. Preferably, the clamp comprises a first pair of clamping arms protruding backwardly from the obturator element and a second pair of actuation arms protruding forwardly from the obturator element. Thus, when actuating the actuation arms, for example by means of a suitable instrument such as clamps, the clamping arms open. It is possible to displace the device until the clamping arms surround the nasotracheal tube in the desired position and then releasing the actuation arms. The force of a spring then causes the clamping arms to close, thus being coupled to the nasotracheal tube.

The attachment element may have clamps in any number and size provided it allows for the device to be coupled to the nasotracheal tube sufficiently firmly to prevent undesired movement thereof. In particular, in a preferred embodiment of the invention, the attachment element comprises an upper clamp in the upper portion of the obturator element and a lower clamp in the lower portion of the obturator element.

### c) Safety element

The purpose of the safety element is providing a clear and evident indication to the medical professionals informing that the obturation device is inside the respiratory airway of the patient. Therefor, the safety element comprises a thread, connected to the obturator element or to the attachment element, having a free end with a clearly visible safety plate. The safety plate is a not necessarily flat part having a size making it easily noticed. In the context of a surgical procedure of this type, the safety plate may have a main dimension of, at least, about two centimetres. Further, in order to make it even more visible, the safety plate may be bright coloured, such as for example red, orange or yellow. The safety plate may further have a warning message written thereon, such as for example "OBTURATION!" or "THROAT PACK!".

In a particularly preferred embodiment, the safety plate is further shaped as an open ring suitable for being placed around the nasotracheal tube outside the patient. Indeed, thanks to being shaped as an open ring or circular crown section having an internal diameter that is larger than the external diameter of the nasotracheal tube, the surgeon can place this ring around the nasotracheal tube emerging outwards through the nose of the patient. Therefore, the safety mechanism is doubled, since the coloured ring indicating the presence of the obturation device needs to be extracted before extubating the patient

Thanks to this configuration, once the obturation device is installed in the respiratory airway of the patient adjacent the oropharynx, the safety plate, joined to the device by the thread, is placed such that it protrudes form the corner of the mouth of the patient. This way, it would be practically impossible to forget that the device is inside the patient, thus reducing greatly the probability of said device being forgotten inside.

In a particularly preferred embodiment of the invention, the device of the invention further comprises a biosensor provided on the surface of the obturator element for measuring vital variables of the patient. Indeed, by using intelligent plastic materials made by functional printing, it is possible to embed biosensors in the obturator element. These biosensors allow for the incorporation of new functions to the device, such as temperature detection, continuous pulse oximetry, cardiac frequency, and others. The biosensors may be connected to the anaesthesia equipment, either by a cabled or wireless connection, thereby providing an additional indication through visual and/or sound alarms in the anaesthesia monitor ensuring the extraction of the obturation device.

The installation method for this new device would be as follows. Firstly, the medical professional grabs the obturation device by means of a suitable instrument, such as clamps. When grabbing the device, the clamps apply a compression force that causes the device to fold, thereby reducing its volume. The medical professional then introduces the clamps through the mouth of the patient, where the patient already has the nasotracheal tube in position, until the obturation device reaches the oropharynx. This operation is carried out ensuring that the safety plate stays outside the mouth of the patient at all times. Once the device is positioned with the desired orientation, with the wall making up the upper portion of the obturator element facing the rear area of the oropharynx and the pair of lobular cavities facing towards the epiglottis and the rear of the tongue, the medical professional opens the clamps and releases the obturation device. Thanks to the shape memory characteristics, the obturation device elastically returns to its original shape, firmly abutting on the oropharynx walls and thus making up a hermetic seal. Then, using the clamp again, the attachment element is actuated for coupling the blocking device to the nasotracheal tube. Finally, the clamp is extracted. The device is installed in position for hermetically sealing the oropharynx of the patient, and the safety place protrudes outwardly through the mouth of the patient for unambiguously indicating that the device is installed, thereby preventing it to be forgotten.

The device of the invention may also comprise a dedicated introductor particularly designed for placing the obturation device in the desired position. According to a preferred embodiment, the introductor is formed by a curved rod configured for being introduced along the respiratory airway of the patient. Further, a distal end of the introductor is couplable to the attachment element of the device to the nasotracheal tube, i.e. when applicable the actuation arms belonging to the upper or lower clamp of the attachment element.

Thus, thanks to this configuration, the medical professional may couple the distal end of the introductor to the upper or lower attachment clamp of the obturation device and, thereafter, introduce the obturation device through the mouth of the patient in a similar way as disclosed above.

The coupling between the obturation device and the introductor may be separable, in which case the introductor is disconnected from the obturation device and extracted from within the patient once the obturation device is installed in position.

Alternatively, the coupling therebetween may be permanent, in which case the introductor remains coupled to the obturation device during the whole surgical procedure. In this second case, the coupling between the distal end of the introductor and the actuation arms of the clamp belonging to the attachment element is articulated. Thus, the medical professional may place the proximal end of the introductor at one or the other side end of the mouth of the patient as required during the surgical procedure.

Further, the obturation device may be disposable.

### BRIEF DESCRIPTION OF THE FIGURES

Fig. 1 shows a traditional obturation device made of gauze and placed in the oropharynx of a patient according to the above disclosed technique.
Fig. 2 shows a front view of a obturation device according to the present invention.
Fig. 3 shows a side view of the obturation device shown in Fig. 2
Fig. 4 shows an upper view of a obturation device shown in Fig. 2
Fig. 5 shows a longitudinal section view of the obturation device placed in the oropharynx of the patient.
Fig. 6 shows a view from the outside of the mouth of the patient of the obturation device placed in the oropharynx of the patient.
Figs. 7a and 7b show respective front and side views of a attachment clamp between the obturation device and the nasotracheal tube.
Fig. 8 shows an exterior view where a safety plate having a ring shape is visible.
Fig. 9 shows a view of the obturation device showing a biosensor embedded in the obturator element.

### PREFERRED EMBODIMENT OF THE INVENTION

An example of the device (1) according to the present invention with reference to the attached drawings is now disclosed.

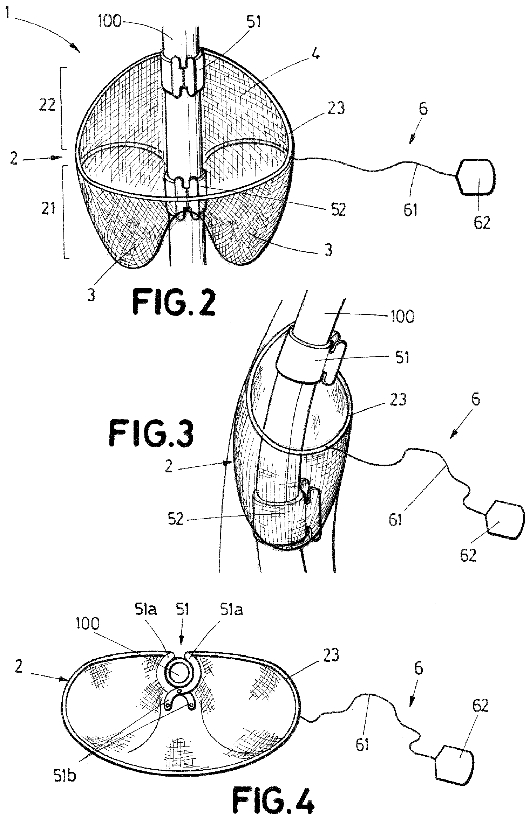

Figs. 2-4 show respective views of the obturation device (1) of the invention illustrating the different component parts thereof. The device (1) comprises a obturator element (2) made of latex-free silicone and configured for being adapted around the nasotracheal tube (100) in the oropharyngeal area of the patient. This obturator element (2) is formed by an upper portion (22) and a lower portion (21), where the lower portion (21) comprises two sac shaped lobular cavities (3) extending essentially downwards and the upper portion (22) comprises an essentially vertical rear wall (2). The two lobular cavities (3) are shaped for adapting perfectly to the front side of the oropharynx, specifically by abutting on the side of the tongue (310) and leaving a gap therebetween where the patient's epiglottis (400) fits. The rear wall (4) protrudes slightly backwards for abutting on the rear side of the oropharynx.

The obturator element (2) has shape memory. That is, in this specific example, the silicone it is made of is somewhat elastic for allowing its shape to change when a particular compression force is applied, and then recovering its original shape disclosed in the above paragraph when said force is no longer applied. Therefor, in this particular example, the obturator element (2) has a peripheric reinforcement (23) along its edge having a relatively higher rigidity in comparison with the rest of the obturator element (2). This peripheric reinforcement (23) ensures that the obturator element (2) as a whole can recover its original shape after being folded through the application of a force. Thanks to this feature, a pneumatic inflation system of the type needed in some known devices is not necessary.

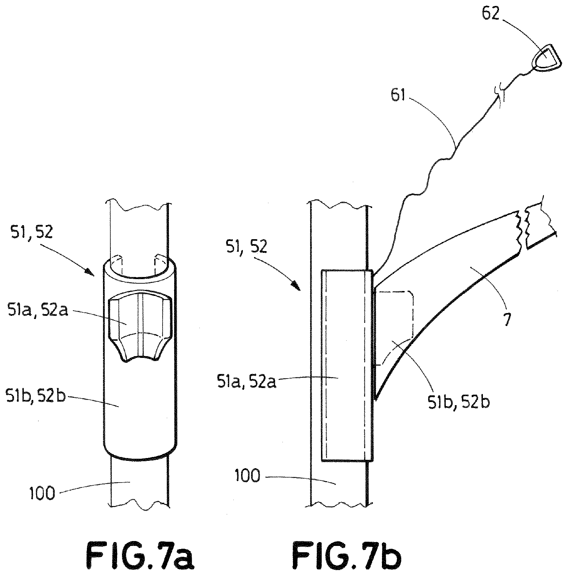

Attachment elements (51, 52), which in this particular example take the form of clamps (51, 52), are permanently joined to the obturator element (2) for coupling it to the nasotracheal tube (100). The attachment elements (51, 52) comprise an upper clamp (51) joined to the rear wall (4) of the upper portion (22) of the obturator element (2) and a lower clamp (52) joined to the lower portion (21) in a place located near the peripheric reinforcement (23) between both lobular cavities (3). Each of the clamps, shown in more detail in Figs. 7a and 7b, has a pair of clamping arms (51a, 52a) protruding backwardly from the obturator element (2), that is, towards the rear side of the oropharynx, and a pair of actuation arms (51b, 52b) protruding forwardly, that is, towards the front side of the oropharynx.

Thanks to this configuration, the device (1) of the invention can be coupled to the nasotracheal tube (100). Therefor, the actuation arms (51b, 52b) of each of the attachment clamps (51, 52) are compressed, thus causing the opening of the respective clamping arms (51a, 52a). Once the clamping arms (51a, 52a) are open, the device (1) is displaced until the desired portion of the nasotracheal tube (100) is clamped by each of the pairs of clamping arms (51a, 52a). Finally, the actuation arms (51b, 52b) are released, a spring mechanism common in conventional clamps thus causing the clamping arms (51a, 52a) to close, thus being firmly coupled to the nasotracheal tube (100). In the final situation, the nasotracheal tube (100) runs vertically along the obturator element (2) essentially in parallel to the rear wall (4). Thus, when the obturator element (2) is placed in position, the patient can breathe through the nasotracheal tube (100), while the obturator element (2) coupled thereto prevents any fluid or foreign element to pass downwardly along the exterior area of the nasotracheal tube (100).

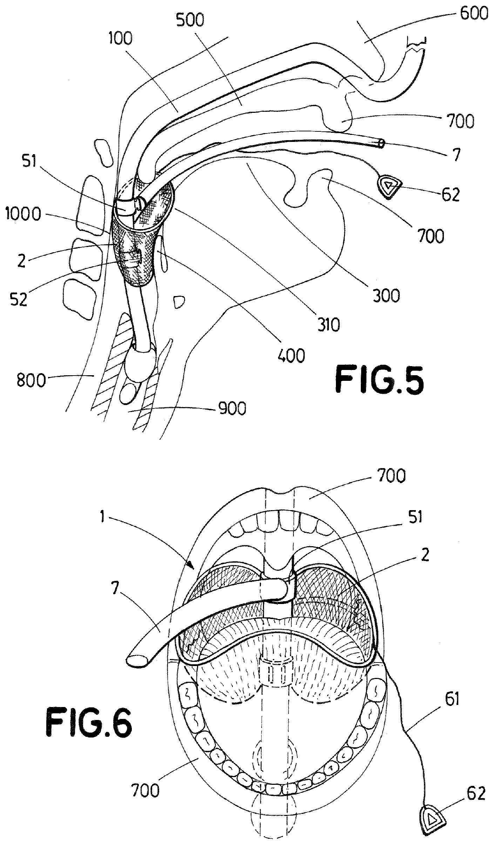

Further, the obturation device (1) of the present invention comprises a safety element (6) designed for preventing the possibility of the medical professional carrying out the surgical procedure forgetting the device (1) inside the patient. The safety element (6) is formed by a thread (61) having an end permanently fixed to the obturator element (2) or to one of the attachment clamps (51, 52) and having, in the other end, a safety plate (62) coloured in red or yellow. The length of the thread (61) is about 10-15 cm, enough for the end where the safety plate (62) is to protrude outwards through the mouth of the patient. The medical professional thus can always see the safety plate (62), which shows that the device (1) of the invention is inside the patient (see Figs. 6 and 7).

The obturation device (1) of the invention can further comprise a particularly designed introductor (7) for facilitating the installation thereof inside the patient's oropharynx, as shown in Figs. 6 and 7. The introductor (7) consists of a rod having a curvature suitable for the introduction thereof, through the patient's mouth, towards the oropharyngeal area of the patient. The distal end of the introductor (7) can be coupled to one of the attachment clamps (51, 52) of the device (1). This coupling can be implemented in several ways, such as for example by means of a pressure attachment. Thus, the medical professional can introduce the device (1) of the invention through the mouth of the patient aided by the introductor (7). Once in position, the introductor (7) can be disconnected from the obturation device (1) and then be extracted. Alternatively, the connection between the introductor (7) and the obturation device (1) can be permanent, in which case the introductor (7) has an articulation (not shown) in an area near its distal end. Thus, the proximal end of the introductor (7) would protrude from the mouth of the patient at all times and, thanks to the aforementioned articulation, it would be possible to select at which side of the mouth is located.

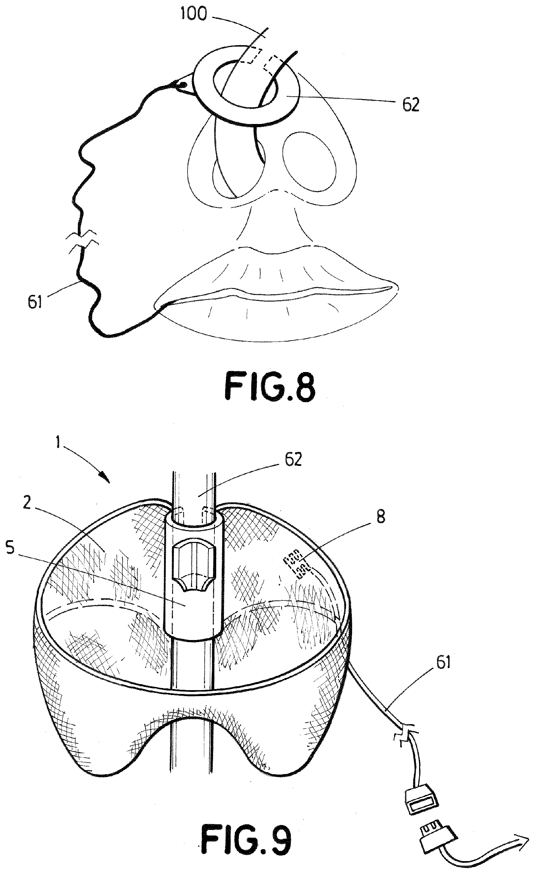

Fig. 8 shows a particular example where the safety plate (62) as an open ring shape whose inner diameter is greater than the exterior diameter of the nasotracheal tube (100). Thanks to the elasticity of the ring, it can be opened and placed around the nasotracheal tube (100). Thus, it is impossible not to notice that the obturation device (1) is installed when extubating the patient

Fig. 8 shows another example of the invention where a single attachment element (5) between the device (1) and the nasotracheal tube (100) is used. As seen, the attachment element (5) has the same configuration as any of the attachment clamps (51, 52) disclosed in connection with Figs. 7a and 7b. Further, Fig. 9 shows a biosensor (8) placed on the surface of the obturator element (2). In this specific case, the biosensor (8) is connected to the monitors by means of an electric cable joined or integrated to the thread (61) of the safety element (6).

## Claims

1. Oropharyngeal obturation device (1) configured for blocking the oropharynx and hypopharynx of a patient during a surgical procedure with nasotracheal intubation, comprising:
- an obturator element (2) having a shape comprising a lower portion (21) and an upper portion (22),
where the lower portion (21) comprises two parallel lobular cavities (3) shaped as two bottom-closed sacs and is configured for adapting to the anatomy of the front portion of the patient's oropharynx such that it abuts on the side of the tongue (310) with the epiglottis (400) housed in a gap separating both lobular cavities (3), and
where the obturator element (2) has shape memory, such that it can be folded during a device (1) introduction procedure and recover its shape once in the oropharyngeal area of the patient, thus sealing the respiratory airway adjacent the oropharynx;
- at least a attachment element (5; 51, 52) joined to the obturator element (2), where the attachment element (5; 51, 52) is configured for being coupled to the nasotracheal tube (100) adjacent the patient's oropharynx; and
- a safety element (6), comprising a thread (61) fixed to the obturator element (2) or to the attachment element (5; 51, 52) and in whose free end a very visible safety plate (62) is present,
the oropharyngeal obturation device (1) being **characterized in that** the upper portion (22) of the obturator element (2) comprises a rear wall (4) protruding from rear edges of the lobular cavities (3) and is configured for adapting to the anatomy of the rear portion of the oropharynx of the patient such that it abuts on the rear wall (1000) of the oropharynx.

2. Device (1) according to claim 1, where the obturator element (2) is made of an elastic material having a peripheric reinforcement (23) along an exterior edge thereof having an elasticity lower than that of the rest of the obturator element (2).

3. Device (1) according to any of the previous claims, where the obturator element (2) is made of a latex-free hypoallergenic material.

4. Device (1) according to claim 3, where the obturator element (2) is made of plastic or silicone.

5. Device (1) according to any of the previous claims, where the attachment element (51, 52) comprises at least a clamp (51, 52) configured for being coupled to the nasotracheal tube (100).

6. Device (1) according to claim 5, where the clamp (51, 52) comprises a first pair of clamping arms (51a, 52a) protruding backwardly from the obturator element (2) and a second pair of actuation arms (51b, 52b) protruding forwardly from the obturator element (2).

7. Device (1) according to any of claims 5-6, where the attachment element (51, 52) comprises an upper clamp (51) at the upper portion (22) of the obturator element (2) and a lower clamp (52) at the lower portion (21) of the obturator element (2).

8. Device (1) according to any of the previous claims, where the safety plate (62) is coloured in bright colours, such that is very visible.

9. Device (1) according to claim 8, where the safety plate (62) is orange, red or yellow.

10. Device (1) according to any of the previous claims, where the safety plate (62) has the shape of an open ring for being placed around the nasotracheal tube (100) outside the patient.

11. Device (1) according to any of the previous claims, further comprising an introductor (7) formed by a curved rod configured for being introduced along the respiratory airway of the patient, where a distal end of the introductor (7) is couplable to the attachment element (51, 52) of the device (1) to the nasotracheal tube (100).

12. Device (1) according to claim 11, where the distal end of the introductor (7) is couplable to the actuation arms (51b, 52b) of the upper or lower clamp (51, 52).

13. Device (1) according to claim 12, where the coupling between the distal end of the introductor (7) and the actuation arms (51b, 52b) of the upper or lower clamp (51, 52) is dismountable.

14. Device (1) according to claim 12, where the coupling of the distal end of the introductor (7) and the actuation arms (51b, 52b) of the attachment clamp (51, 52) is permanent.

15. Device (1) according to claim 14, where the introductor (7) comprises an articulation adjacent its distal end.

16. Device (1) according to any of the previous claims, further comprising a biosensor (8) on the surface of the obturator element (2) for measuring vital variables of the patient.

17. Device (1) according to any of the previous claims, that is disposable.

## Patentansprüche

1. Oropharyngeale Verschlussvorrichtung (1), die zum Blockieren des Oropharynx und des Hypopharynx eines Patienten während eines chirurgischen Eingriffs mit nasotrachealer Intubation konfiguriert ist, umfassend:
- ein Verschlusselement (2) mit einer Form, die einen unteren Abschnitt (21) und einen oberen Abschnitt (22) umfasst,
wobei der untere Abschnitt (21) zwei parallele lobuläre Hohlräume (3) umfasst, die als zwei unten geschlossene Beutel geformt sind, und konfiguriert ist, um sich an die Anatomie des vorderen Abschnitts des Oropharynx des Patienten anzupassen, sodass er an der Seite der Zunge (310) anliegt, wobei die Epiglottis (400) in einem Spalt untergebracht ist, der beide lobulären Hohlräume (3) trennt, und
wobei das Verschlusselement (2) ein Formgedächtnis aufweist, so dass es während eines Einführungsvorgangs der Vorrichtung (1) gefaltet werden kann und seine Form nach der Einführung in den oropharyngealen Bereich des Patienten wiederherstellen kann, wodurch der Atemweg neben dem Oropharynx abgedichtet wird;
- mindestens ein Befestigungselement (5; 51, 52), das mit dem Verschlusselement (2) verbunden ist, wobei das Befestigungselement (5; 51, 52) dazu konfiguriert ist, an den Nasotrachealtubus (100) angrenzend an den Oropharynx des Patienten gekoppelt zu werden; und
- ein Sicherheitselement (6), das ein Gewinde (61) umfasst, das an dem Verschlusselement (2) oder an dem Befestigungselement (5; 51, 52) befestigt ist und an dessen freiem Ende eine gut sichtbare Sicherheitsplatte (62) vorhanden ist,
wobei die oropharyngeale Verschlussvorrichtung (1) **dadurch gekennzeichnet ist, dass** der obere Abschnitt (22) des Verschlusselements (2) eine Rückwand (4) umfasst, die von Hinterkanten der lobulären Hohlräume (3) vorsteht und konfiguriert ist, um sich an die Anatomie des hinteren Abschnitts des Oropharynx des Patienten anzupassen, sodass er an der Rückwand (1000) des Oropharynx anliegt.

2. Vorrichtung (1) nach Anspruch 1, wobei das Verschlusselement (2) aus einem elastischen Material hergestellt ist, das eine periphere Verstärkung (23) entlang eines äußeren Randes davon aufweist, die eine geringere Elastizität als die des übrigen Verschlusselements (2) aufweist.

3. Vorrichtung (1) nach einem der vorhergehenden Ansprüche, wobei das Verschlusselement (2) aus einem latexfreien hypoallergenen Material hergestellt ist.

4. Vorrichtung (1) nach Anspruch 3, wobei das Verschlusselement (2) aus Kunststoff oder Silikon hergestellt ist.

5. Vorrichtung (1) nach einem der vorhergehenden Ansprüche, wobei das Befestigungselement (51, 52) mindestens eine Klemme (51, 52) umfasst, die konfiguriert ist, um mit dem Nasotrachealtubus (100) gekoppelt zu werden.

6. Vorrichtung (1) nach Anspruch 5, wobei die Klemme (51, 52) ein erstes Paar von Klemmarmen (51a, 52a), die von dem Verschlusselement (2) nach hinten vorstehen, und ein zweites Paar von Betätigungsarmen (51b, 52b), die von dem Verschlusselement (2) nach vorne vorstehen, umfasst.

7. Vorrichtung (1) nach einem der Ansprüche 5 bis 6, wobei das Befestigungselement (51, 52) eine obere Klemme (51) am oberen Abschnitt (22) des Verschlusselements (2) und eine untere Klemme (52) am unteren Abschnitt (21) des Verschlusselements (2) umfasst.

8. Vorrichtung (1) nach einem der vorhergehenden Ansprüche, wobei die Sicherheitsplatte (62) eine helle Farbe hat, so dass sie gut sichtbar ist.

9. Vorrichtung (1) nach Anspruch 8, wobei die Sicherheitsplatte (62) orange, rot oder gelb ist.

10. Vorrichtung (1) nach einem der vorhergehenden Ansprüche, wobei die Sicherheitsplatte (62) die Form eines offenen Rings hat, um außerhalb des Patienten um den Nasotrachealtubus (100) herum angeordnet zu werden.

11. Vorrichtung (1) nach einem der vorhergehenden Ansprüche, die des Weiteren eine Einführungsvorrichtung (7) umfasst, die durch einen gekrümmten Stab gebildet ist, der so konfiguriert ist, dass er entlang des Atemwegs des Patienten eingeführt werden kann, wobei ein distales Ende der Einführungsvorrichtung (7) mit dem Befestigungselement (51, 52) der Vorrichtung (1) mit dem Nasotrachealtubus (100) koppelbar ist.

12. Vorrichtung (1) nach Anspruch 11, wobei das distale Ende der Einführungsvorrichtung (7) mit den Betätigungsarmen (51b, 52b) der oberen oder unteren Klemme (51, 52) koppelbar ist.

13. Vorrichtung (1) nach Anspruch 12, wobei die Kopplung zwischen dem distalen Ende der Einführungsvorrichtung (7) und den Betätigungsarmen (51b, 52b) der oberen oder unteren Klemme (51, 52) demontierbar ist.

14. Vorrichtung (1) nach Anspruch 12, wobei die Kopplung des distalen Endes der Einführungsvorrichtung (7) und der Betätigungsarme (51b, 52b) der Befestigungsklemme (51, 52) permanent ist.

15. Vorrichtung (1) nach Anspruch 14, wobei die Einführungsvorrichtung (7) angrenzend an ihr distales Ende ein Gelenk aufweist.

16. Vorrichtung (1) nach einem der vorhergehenden Ansprüche, die des Weiteren einen Biosensor (8) auf der Oberfläche des Verschlusselements (2) zum Messen von Vitalparametern des Patienten umfasst.

17. Vorrichtung (1) nach einem der vorhergehenden Ansprüche, die zum einmaligen Gebrauch bestimmt ist.

## Revendications

1. Dispositif de tamponnade oropharyngée (1) configuré pour bloquer l'oropharynx et l'hypopharynx d'un patient pendant une intervention chirurgicale avec intubation nasotrachéale, comprenant :
- un élément obturateur (2) ayant une forme comprenant une partie inférieure (21) et une partie supérieure (22),
où la partie inférieure (21) comprend deux cavités lobulaires parallèles (3) en forme de deux sacs fermés par le bas et elle est configurée pour s'adapter à l'anatomie de la partie avant de l'oropharynx du patient de telle sorte qu'elle bute sur le côté de la langue (310) avec l'épiglotte (400) logée dans un espace séparant les deux cavités lobulaires (3), et
lorsque l'élément obturateur (2) a une mémoire de forme telle qu'il peut être plié pendant une procédure d'introduction du dispositif (1) et retrouver sa forme une fois dans la zone oropharyngée du patient, fermant ainsi les voies respiratoires adjacentes à l'oropharynx ;
- au moins un élément de fixation (5 ; 51, 52) relié à l'élément obturateur (2), l'élément de fixation (5 ; 51, 52) étant configuré pour être couplé au tube nasotrachéal (100) adjacent à l'oropharynx du patient ; et
- un élément de sécurité (6), comprenant un filetage (61) fixé à l'élément obturateur (2) ou à l'élément de fixation (5 ; 51, 52) et à l'extrémité libre duquel se trouve une plaque de sécurité (62) très visible,
le dispositif de tamponnade oropharyngée (1) étant **caractérisé en ce que** la partie supérieure (22) de l'élément obturateur (2) comprend une paroi arrière (4) faisant saillie à partir des bords arrière des cavités lobulaires (3) et est configurée pour s'adapter à l'anatomie de la partie arrière de l'oropharynx du patient de telle sorte qu'elle bute sur la paroi arrière (1000) de l'oropharynx.

2. Dispositif (1) selon la revendication 1, dans lequel l'élément obturateur (2) est réalisé en un matériau élastique ayant un renforcement périphérique (23) le long d'un bord extérieur de celui-ci ayant une élasticité inférieure à celle du reste de l'élément obturateur (2).

3. Dispositif (1) selon l'une quelconque des revendications précédentes, dans lequel l'élément obturateur (2) est constitué d'un matériau hypoallergénique exempt de latex.

4. Dispositif (1) selon la revendication 3, dans lequel l'élément obturateur (2) est en matière plastique ou en silicone.

5. Dispositif (1) selon l'une quelconque des revendications précédentes, dans lequel l'élément de fixation (51, 52) comprend au moins une pince (51, 52) configurée pour être couplée au tube nasotrachéal (100).

6. Dispositif (1) selon la revendication 5, dans lequel la pince (51, 52) comprend une première paire de bras de serrage (51a, 52a) faisant saillie vers l'arrière à partir de l'élément obturateur (2) et une seconde paire de bras d'actionnement (51b, 52b) faisant saillie vers l'avant à partir de l'élément obturateur (2).

7. Dispositif (1) selon l'une quelconque des revendications 5 à 6, dans lequel l'élément de fixation (51, 52) comprend une bride supérieure (51) au niveau de la partie supérieure (22) de l'élément obturateur (2) et une bride inférieure (52) au niveau de la partie inférieure (21) de l'élément obturateur (2).

8. Dispositif (1) selon l'une quelconque des revendications précédentes, dans lequel la plaque de sécurité (62) est colorée dans des couleurs vives, de sorte qu'elle est très visible.

9. Dispositif (1) selon la revendication 8, dans lequel la plaque de sécurité (62) est orange, rouge ou jaune.

10. Dispositif (1) selon l'une quelconque des revendications précédentes, dans lequel la plaque de sécurité (62) a la forme d'un anneau ouvert destiné à être placé autour du tube nasotrachéal (100) à l'extérieur du patient.

11. Dispositif (1) selon l'une quelconque des revendications précédentes, comprenant en outre un introducteur (7) formé par une tige incurvée configurée pour être introduite le long des voies respiratoires du patient, où une extrémité distale de l'introducteur (7) peut être couplée à l'élément de fixation (51, 52) du dispositif (1) au tube nasotrachéal (100).

12. Dispositif (1) selon la revendication 11, dans lequel l'extrémité distale de l'introducteur (7) peut être couplée aux bras d'actionnement (51b, 52b) de la pince supérieure ou inférieure (51,52).

13. Dispositif (1) selon la revendication 12, dans lequel le couplage entre l'extrémité distale de l'introducteur (7) et les bras d'actionnement (51b, 52b) de la pince supérieure ou inférieure (51, 52) est démontable.

14. Dispositif (1) selon la revendication 12, dans lequel l'accouplement de l'extrémité distale de l'introducteur (7) et des bras d'actionnement (51b, 52b) de la pince de fixation (51, 52) est permanent.

15. Dispositif (1) selon la revendication 14, dans lequel l'introducteur (7) comprend une articulation adjacente à son extrémité distale.

16. Dispositif (1) selon l'une quelconque des revendications précédentes, comprenant en outre un biocapteur (8) sur la surface de l'élément obturateur (2) pour mesurer des variables vitales du patient.

17. Dispositif (1) selon l'une quelconque des revendications précédentes, qui est jetable.
